# EUROPEAN PATENT SPECIFICATION

(11) **EP 0 639 079 B1**
(45) Date of publication and mention of the grant of the patent: **12.01.2000**
(21) Application number: 93910992.2
(22) Date of filing: 28.04.1993
(51) Int. Cl.: A61K 38/00, A61K 38/21

(54) **METHODS FOR TREATING INTERLEUKIN-1 AND TUMOR NECROSIS FACTOR MEDIATED DISEASES**
Methoden zur Behandlung von Interleukin- 1 und - Tumor - Nekrose - Faktor - verursachten Krankheiten
METHODES DE TRAITEMENT DES MALADIES INDUITES PAR L'INTERLEUKINE-1 ET LE FACTEUR DE NECROSE TUMORALE

(30) Priority: 30.04.1992 US 876344
(43) Date of publication of application: 22.02.1995
(73) Proprietor: Amgen Inc., Thousand Oaks, CA 91320-1799 (US)
(72) Inventor: THOMPSON, Robert, C., Boulder, CO 80303 (US); RUSSELL, Deborah, A., Boulder, CO 80303 (US)
(74) Representative: Grünecker, Kinkeldey, Stockmair & Schwanhäusser Anwaltssozietät
(86) International application number: US9304141
(87) International publication number: WO9321946

(56) References cited:
- EP-A- 0 343 684
- EP-A- 0 418 014
- EP-A- 0 422 339
- WO-A-92/13095
- THE JOURNAL OF RHEUMATOLOGY , VOL 19 SUPPL-32 January 1992, pages 59 - 60 J-M. DAYER 'Immunomodulating functions of tumor necrosis factor and interleukin 1 inhibitors'

## Description

### BACKGROUND OF THE INVENTION

The present invention relates generally to a pharmaceutical composition for preventing and treating diseases, and more particularly, to a pharmaceutical composition for preventing and treating certain Tumor Necrosis Factor (TNF) mediated diseases and Interleukin-1 (IL-1) mediated diseases.

Inflammation is the body's defense reaction to injury such as those caused by mechanical damage, infection, or antigenic stimulation. An inflammatory reaction may be expressed pathologically when inflammation is induced by an inappropriate stimulus such as an autoantigen, expressed in an exaggerated manner, or persists well after the removal of the injurious agents. Under these conditions, inflammation may be expressed chronically. The mediation of acute inflammatory diseases such as septic shock and chronic inflammatory diseases such as rheumatoid arthritis and inflammatory bowel disease has been linked to the proinflammatory activities of IL-1 and TNF.

TNF and IL-1 are naturally occurring compounds that are often referred to as cytokines. Cytokines are extacellular proteins that modify the behavior of cells, particularly those cells that are in the immediate area of cytokine synthesis and release.

One of the most potent inflammatory cytokines yet discovered and a cytokine which is thought to be a key mediator in many diseases and medical conditions is IL-1. IL-1, which is manufactured, though not exclusively, by cells of the macrophage/monocyte lineage, may be produced in two forms, IL-1 alpha (Il-1α) and IL-1 beta (IL-1β).

Tumor Necrosis Factors (TNFs) are a class of cytokines produced by numerous cell-types, including monocytes and macrophages. At least two TNFs have been previously described, specifically TNF alpha (TNFα) and TNF beta (TNFβ or lymphotoxin). These known TNFs have important physiological effects on a number of different target cells involved in the inflammatory response. The proteins cause both fibroblasts and synovial cells to secrete latent collagenase and prostaglandin E2, and cause osteocyte cells to stimulate bone resorption. These proteins increase the surface adhesive properties of endothelial cells for neutrophils. They also cause endothelial cells to secrete coagulant activity and reduce their ability to lyse clots. In addition they redirect the activity of adipocytes away from the storage of lipids by inhibiting expression of the enzyme lipoprotein lipase.

TNFs cause hepatocytes to synthesize a class of proteins known as "acute phase reactants," which act on the hypothalamus as pyrogens. Through these activities, it has been seen that TNFs play an important part in an organism's response to a variety of indications such as infection and injury. See, e.g., articles by P.J. Selby et al., Lancet, February 27, 1988, pg. 483; H.F. Starnes, Jr. et al., J. Clin Invest., vol. 82, pg. 1321 (1988); A. Oliff et al. Cell, vol. 50, pg. 555 (1987); and A. Waage et al., Lancet, February 14, 1987, pg. 355.

Activities of both IL-1 and TNF are detected in rum and other body fluids sampled in human inflammatory diseases. In experimental systems, after a short-term injurious stimulus, the kinetics of the release of IL-1 and TNF into the circulation follows a well-characterized pattern. A bolus administration of the bacterial product, lipopolysaccharide (LPS), to baboons causes TNF and IL-1 to be synthesized and released sequentially peaking at 2 and 6 hours, respectively, post challenge. The cellular source of IL-1 and TNF differs. Whereas TNF is primarily synthesized only by monocytes, IL-1 can be produced by virtually all nucleated cell types. In the early stages of inflammatory reactions, however, the cells most likely involved in the production of IL-1 are epithelial and endothelial cells and cells of the monocyte/macrophage lineage.

The synthesis of IL-1 and TNF is stimulated in common by bacterial products, lectins, immune complexes, and a variety of noxious stimuli which produce tissue damage. IL-1 and TNF share the following proinflammatory activities: (1) increase the adherence of neutrophils to vascular endothelium, (2) activate the respiratory burst in neutrophils and monocytes, (3) stimulate resorption of cartilage matrix and bone, (4) stimulate prostaglandin release from and proliferation of a number of cell types, and (5) stimulate fever, cachexia, anorexia and acute phase proteins.

In addition to the shared inflammatory properties, the observations that (1) IL-1 and TNF are synthesized in response to the same inflammatory stimuli and that (2) TNF itself induces the release of IL-1 indicate that a close relationship exists between the two cytokine systems. In fact, the synergistic effects of combined stimulation with IL-1 and TNF have been observed in many experimental systems of inflammation. A synergistic effect is defined as occurring when the effect of two agents in combination is greater than the algebraic sum of their individual effects. For example, the injection of submaximal doses of IL-1 and TNF into the rabbit knee joint resulted in a marked synergy with respect to the accumulation of polymorphonuclear neutrophils (Henderson, B., Clin. Exp. Immunol. 75:306-310 (1989)). In addition, the combined administration to mice of IL-1 and TNF at doses which are individually sublethal causes the synergistic induction of a septic shock-like condition which is lethal to 100% of the mice (Everaerdt, B., Biochem. Biophys. Res. Commun., 163:378 (1989); Waage et al., J. Exp. Med. 167:1987-1992 (1988)). Therefore, if in disease states both IL-1 and TNF are required to elicit a full pathological response via a synergistic interaction, then it was believed that the administration of either an IL-1 inhibitor or a TNF inhibitor would be sufficient to maximally inhibit an inflammatory response.

EP-A-0422339 discloses TNF inhibitors as useful therapeutics against septic shock and cachexia. This document further discloses that TNF inhibitors could be even more effective when administered in conjunction with interleukin-1 inhibitors. A specific IL-1 inhibitor is not mentioned in EP-A-0422339.

EP-A-0418014 discloses tumor necrosis factor receptor proteins for suppressing TNF dependent inflammatory responses. It is further disclosed that a combination therapy using both IL-1 receptors or IL-2 receptors may be preferred in the treatment of TNF-associated clinical indications.

### SUMMARY OF THE INVENTION

The surprising and unexpected result discovery relaxing to the present invention is the ability of an IL-1 inhibitor and a TNF inhibitor to act not only additively, but also synergistically in some cases in the teaxinent of IL-1-mediated and TNF-mediated diseases. Thus, the present invention relates to a pharmaceutical composition comprising IL-1 inhibitor IL-1ra and a TNF inhibitor in a pharmaceutically acceptable carrier. More particularly, the present invention relates to a pharmaceutical composition for the treatment of certain diseases and medical conditions - many of which can be characterized as inflammatory diseases - that are mediated by both IL-1 and TNF. Among the indications that may be treated with the composition of the present invention are septic shock, arthritis, inflammatory bowel disease, adult respiratory distress syndrome, pulmonary fibrosis, ischemic injury and reperfusion injury.

TNF inhibitors that can be used in the present invention are naturally-occurring proteins and truncated forms of naturally-occurring proteins. The naturally occurring proteins are useful because they pose a relatively low risk of producing unforseen side effects in patients treated therewith. In other embodiments of this invention, muteins of protein TNF inhibitors where only a small number of amino acid residues differ from the natural protein sequence are also referred to as TNF inhibitors.

The TNF inhibitors can be soluble fragments of TNF receptor proteins. TNF inhibitors that are useful in the present invention include human TNF binding proteins (TNFbp), particularly soluble fragments of TNF receptor proteins, including, for example, the 30kDa TNF inhibitor and the 40kDa TNF inhibitor. The 40kDa TNF inhibitor can be the full-length 40kDa TNF inhibitor or the truncated forms 40kDa TNF inhibitor Δ51 and 40kDa TNF Inhibitor Δ53. Also useful are proteins that have been modified, for example, by the addition of polyethylene glycol (PEG) or any other repeat polymer to increase their circulating half-life and/or to decrease immunogenicity. TNF inhibitors that act as receptor antagonists to TNF are also included within the scope of this invention.

While the production of TNF inhibitors may be achieved by extraction from naturally available sources, such as by isolation from human urine, a preferred method of TNF inhibitor production is by recombinant DNA technology. Recombinant DNA technology is preferred in part because it is capable of producing comparatively higher amounts of TNF inhibitors at greater purities.

Additional TNF inhibitors include muteins of 30kDa TNF inhibitor wherein selected amino acids of 30kDa TNF inhibitor are replaced with cysteine. Such muteins may be used as TNF inhibitors, or they may be reacted with polyethylene glycol (PEG) to form TNF inhibitor PEG compounds, containing one or two TNF inhibitors per molecule. In a preferred embodiment, the TNF inhibitor is a bivalent species formed in a reaction between a mutein 30kDa TNF inhibitor and a bifunctionalized PEG precursor. The preferred mutein is C105 30kDa TNF inhibitor, where residue 105 of 30kDa TNF inhibitor is replaced with a cysteine residue.

The IL-1 inhibitors useful in the present invention are proteins and, more particularly, are naturally-occurring proteins. The naturally-occurring proteins are particularly useful because they pose a relatively low risk of producing unforeseen side effects in patients treated therewith.

A useful class of interleukin-1 inhibitors is a human protein that act as a natural interleukin-1 receptor antagonist (IL-1ra). Preferably, the IL-1ra that is preferred in the practice of the present invention is selected from the group consisting of IL-1raα, IL-1raβ, IL-1rax, or the N-terminal methionyl derivatives of these IL-1ra. Also preferred are proteins which have been modified for example by the addition of polyethylene glycol (PEG) or any other repeat polymer to increase their circulating half-life and/or to decrease their immunogenicity.

While the production of IL-1ra may be achieved by extraction from naturally available sources, such as by isolation from the conditioned medium of cultured human monocytes, a preferred method of IL-1ra production is by recombinant DNA technology.

Recombinant DNA technology is preferred in part because it is capable of producing comparatively higher amounts of IL-1ra at greater purities.

### BRIEF DESCRIPTION OF THE DRAWINGS

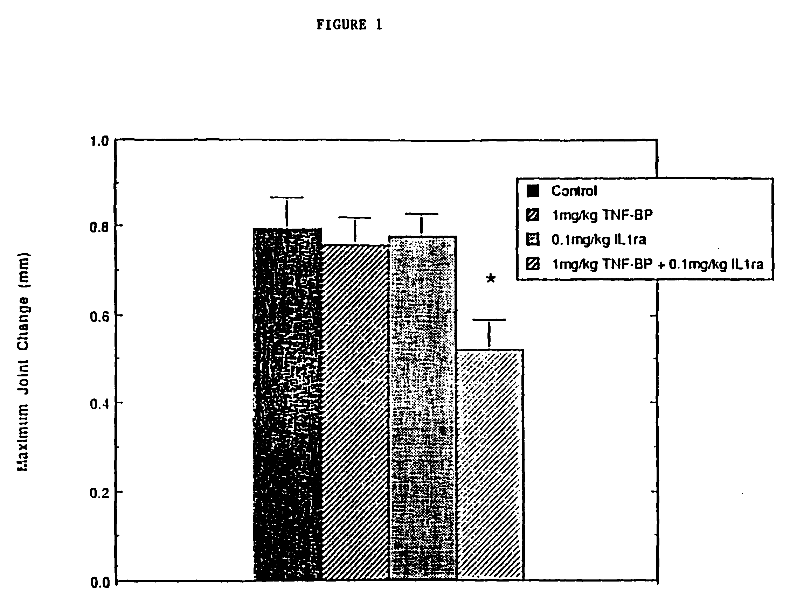

Figure 1 depicts the maximum increases in joint diameter during the 72-hour period after LPS-induced reactivation (* = the significant difference between TNFbp + IL-1ra vs. Vehicle at p <0.025; TNFbp + IL-1ra vs. TNFbp at p < 0.025; and TNFbp + IL-1ra vs. IL-1ra at p < 0.010 by the unpaired t test).

### DETAILED DESCRIPTION OF THE INVENTION

As noted above, the present invention relates to a pharmaceutical composition for preventing and treating IL-1-mediated and TNF-mediated diseases in patients suffering therefrom. A use of this composition comprises the manufacture of a medicament for administration of therapeutically effective amounts of TNF inhibitor and IL-1 inhibitor IL-1ra to a patient suffering from a TNF or IL-1 mediated disease. Although not limited by theory, the interrelationship known to exist between TNF and IL-1 suggests that most, if not all, TNF mediated diseases would also be found to be IL-1 mediated diseases and visa versa.

As described above, it is known that TNF inhibitors may successfully be employed to prevent or treat TNF mediated diseases, and that IL-1 inhibitors may successfully be employed to prevent or teat IL-1 mediated diseases. The surprising and unexpected result discovered by the inventors of the present invention is the ability of the IL-1 inhibitor and the TNF inhibitor to act synergistically in the tearment of IL-1 mediated and TNF mediated diseases. "Synergistically" is used herein to refer to a situation where the benefit conveyed by the joint administration of inhibitors is greater than the algebraic sum of the effects resulting from the separate administration of the components of the combination.

While the present invention relates to a composition for preventing and treating human diseases, veterinary uses an also included within the scope of this invention since guidance is provided for general physiological use.

A disease or medical condition is to be considered to be a "TNF-mediated disease" if the spontaneous or experimental disease is associated with elevated levels of TNF in bodily fluids or in tissues adjacent the focus of the disease or indication within the body. TNF mediated diseases may also be recognized by the following two conditions: (1) pathological findings associated with a disease can be mimicked experimentally in animals by the administration of TNF; and (2) the pathology induced in experimental animal models of the disease can be inhibited or abolished by treatment with agents which inhibit the action of TNF. Many TNF-mediated diseases satisfy two of these three conditions and others will satisfy all three conditions. A non-exclusive list of TNF-mediated diseases includes adult respiratory distress syndrome, pulmonary fibrosis, arthritis, inflammatory bowel disease and septic shock.

A disease or medical condition is considered to be an "interleukin-1 mediated disease" if the spontaneous or experimental disease or medical condition is associated with elevated levels of IL-1 in bodily fluids or tissue or if cells or tissues taken from the body produce elevated levels of IL-1 in culture. In many cases, such interleukin-1 mediated diseases are also recognized by the following additional two conditions: (1) pathological findings associated with the disease or medical condition can be mimicked experimentally in animals by the administration of IL-1; and (2) the pathology induced in experimental animal models of the disease or medical condition can be inhibited or abolished by treatment with agents which inhibit the action of IL-1. In most "interleukin-1 mediated diseases" at least two of the three conditions are met, and in many "interleukin-1 mediated diseases" all three conditions are met. A list of diseases or medical conditions which are interleukin-1 mediated includes, but is not limited to arthritis, inflammatory bowel disease, septic shock, ischemic injury, reperfusion injury, osteoporosis, asthma, insulin diabetes, myelogenous and other leukemia, psoriasis and cachexia/anorexia.

Naturally-occurring inhibitor proteins are preferred in part because they pose a comparatively low risk of producing unforeseen and undesirable physiological side effects in patients treated therewith.

For purposes of the specification and claims, a protein is deemed to be "naturally-occurring" if it or a substantially equivalent protein can be found to exist normally in healthy humans. "Naturally-occurring" proteins specifically includes forms of proteins found to exist in healthy humans that are partially truncated at the carboxyl terminus of such proteins, as well as nonglycosylated forms of proteins that exist in glycosylated forms in healthy humans. "Naturally-occurring" proteins may be obtained by recombinant DNA methods as well as by isolation from cells which ordinarily produce them. "Naturally-occurring" also encompasses proteins that contain an N-terminal methiorryl group as a consequence of expression in E. coli.

"Substantially equivalent" as used throughout the specification and claims is defined to mean possessing a very high degree of amino acid residue homology (See generally M. Dayhoff, Atlas of Protein Sequence and Structure, vol. 5, p. 124 (1972), National Biochemical Research Foundation, Washington, D.C., specifically incorporated herein by reference) as well as possessing comparable biological activity.

Among the TNF inhibitors useful in the present invention are the naturally-occurring proteins that exist in vivo as binding proteins of TNF that are described in EP Application No. 90 113 673.9 entitled "Tumor Necrosis Factor (TNF) Inhibitor and method for Obtaining the Same", filed July 17, 1990.

There are two distinct forms of preferred TNF inhibitors, each disclosed and described in EP Application No. 90 113 673.9. The first of these is the 30kDa TNF inhibitor, which has been identified and isolated from medium conditioned by human U937 cells and from human urine. The 30kDa TNF inhibitor is approximately 30 kDa on SDS-PAGE, and elutes from a DEAE CL6B column at about 80 millimolar NaCl in Tris buffer, pH 7.5. The 30kDa TNF inhibitor has been shown to inhibit the activity of TNF alpha and has little effect on the activity of TNF beta The naturally occurring protein is glycosylated. Nonglycosylated 30kDa TNF inhibitor also exhibits TNF inhibitory activity.

The second form of TNF inhibitors is 40kDa TNF inhibitor, which has also been identified and isolated from at least a medium conditioned by human U937 cells and human urine. The full-length 40kDa TNF inhibitor is a glycoprotein which runs at approximately 40 kDa on SDS-PAGE, and elutes from a DEAE column at about 100 millimolar NaCl in Tris buffer, pH 7.5. This 40kDa TNF inhibitor has been shown to inhibit the activity of both TNF alpha and TNF beta The nonglycosylated protein exhibit TNF inhibitory activity.

Three forms of the 40kDa TNF inhibitor have been recombinantly produced by expression in E. coli. Each of these forms, referred to as full-length 40kDa TNF inhibitor, 40kDa TNF inhibitor Δ51 and 40kDa TNF inhibitor Δ53 (along with the glycosylated full-length 40kDa TNF inhibitor as isolated from medium conditioned by human U937 cells and human urine, in glycosylated and nonglycosylated forms, all of which are collectively referred to herein as 40kDa TNF inhibitor) are described in EP Application No. 90 113 673.9. The Δ51 protein is a truncated version of the native protein wherein 51 amino acid residues at the carboxyl terminus of the mature protein are removed. The Δ53 protein is a truncated version of the mature protein wherein 53 amino acid residues at the carboxyl terminus of the native protein are removed. Naturally-occurring 40kDa TNF inhibitor (glycosylated), and the nonglycosylated inhibitors (native full length, Δ51 and Δ53) have substantially the same TNF inhibitory activity.

The nucleic acid sequences of the genes encoding both the 30kDa TNF inhibitor and the 40kDa TNF inhibitors and the amino acid sequences of these proteins are given in the EP Application No. 90 113 673.9. The present invention encompasses nonglycosylated forms of the TNF inhibitors as well as certain truncated forms of the naturally-occurring proteins as described above. In a further embodiment, the TNF inhibitors are modified by attachment of one or more polyethylene glycol (PEG) or other repeating polymeric moieties as described below.

Methods for producing the TNF inhibitors are also disclosed in EP Application No. 90 113 673.9. One disclosed method involves isolating the inhibitors from various sources, such as human urine and medium conditioned by human U937 cells. A second disclosed method involves isolating the genes responsible for coding the inhibitors, cloning the gene in suitable vectors and cell types, and expressing the gene to produce the inhibitors. The latter method, which is exemplary of recombinant DNA methods in general, is a preferred method of the present invention. Recombinant DNA methods are preferred in part because they are capable of achieving comparatively higher amounts at greater purities.

Preferably, the above described TNF inhibitors are produced by the aforementioned method in "substantially pure" form. By "substantially pure" it is meant that the inhibitor, in an unmodified form, has a comparatively high specific activity. It is to be recognized, however, that derivatives of TNF inhibitors may have different specific activities.

Additional TNF inhibitors include compounds capable of competing with TNF for TNF receptor sites. Such compounds include receptor antagonists. Other TNF inhibitors include compounds and proteins which block in vivo synthesis or extracellular release of TNF. Such compounds include agents which affect transcription or translation of TNF genes or processing of TNF preproteins.

Particularly preferred IL-1ra's of the present invention are the naturally-occurring proteins that exist in vivo as regulators of interleukin-1 that have previously been described in United States Patent No. 5,075,222 by Hannum et al., which is entitled "Interleukin-1 Inhibitors." This U.S. Patent, is referred to herein as the '222 patent.

Three preferred forms of IL-1ra, each being derived from the same DNA coding sequence, are disclosed and described in the '222 parent. The first of these, IL-1raα, is a 22-23 kDa molecule on SDS-PAGE with an approximate isoelectric point of 4.8, during from a Mono Q FPLC column at around 52 mM NaCl in Tris buffer, pH 7.6. The second, IL-1raβ, is a 22-23 kDa protein, p.I=4.8, eluting from a Mono Q column at 60 mM NaCl. The third, IL-1rax, is a 20 kDa protein, eluting from a Mono Q column at 48 mM NaCl. All three of these interleukin-1 inhibitors possess similar functional and immunological activities. The present invention also includes modified IL-1ra's. In one embodiment, the IL-1ra is modified by attachment of one or more polyethylene glycol (PEG) or other repeating polymeric moieties as described below. In another embodiment, the IL-1ra contains an N-terminal methionyl group as a consequence of expression in E. coli.

Methods for producing these IL-1 inhibitors, particularly IL-1ra's, are also disclosed in the '222 patent. One disclosed method involves isolating the inhibitors from human monocytes (where they are naturally produced). A second disclosed method involves isolating the gene responsible for coding the inhibitors, cloning the gene in suitable vectors and cell types, expressing the gene to produce the inhibitors and harvesting the inhibitors. The latter method, which is exemplary of recombinant DNA methods in general, is a preferred method of the present invention. Recombinant DNA methods are preferred in part because they are capable of achieving comparatively higher amounts at greater purities.

Additional interleukin-1 inhibitors include compounds capable of specifically preventing activation of cellular receptors to IL-1. Such compounds include IL-1 binding proteins such as soluble receptors and monoclonal antibodies. Such compounds also include receptor antagonists and monoclonal antibodies to the receptors.

A second class of IL-1ra's include the compounds and proteins which block in vivo synthesis and/or extracellular release of IL-1. Such compounds include agents which affect transcription of IL-1 genes or processing IL-1 preproteins. Under certain conditions the IL-1ra will block IL-1 induced IL-1 production.

Preferably, the above described IL-1ra's are produced by the aforementioned method in "substantially pure" form. By "substantially pure" it is meant that the inhibitor, in an unmodified form, has a comparatively high specific activity, preferably in the range of approximately 150,000-500,000 receptor units/mg as defined by Hannum et al. in Nature 343:336-340 (1990) and Eisenberg et al. in Nature 343:341-346 (1990). It is to be recognized, however, that derivatives of IL-1ra may have different specific activities.

Also included within the scope of this invention are modified TNF and IL-1 inhibitors. The modified inhibitors include, for example, muteins of such inhibitors in which a cysteine residue is substituted for an amino acid at one or more sites in the amino acid sequence of a naturally-occurring inhibitor. Such muteins may then be site-selectively reacted with functionalized polyethylene glycol (PEG) units or other sulfhydryl-containing polyethers to create TNF inhibitor PEG species or IL-1 inhibitor PEG species. PCT Publication No. WO 92/1622 discloses a number of modified TNF and IL-1 inhibitor species and methods of making such PEG modified inhibitors. A 30kDa TNF inhibitor mutein, in which the asparagine at position 105 of 30kDa TNF inhibitor is replaced with cysteine (referred to herein as the "C105 mutein" or "C105"), is particularly useful. In one further embodiment, the mutein proteins may be reacted with bifunctionalized PEG units to form bivalent "dumbbell" species wherein two cytokine species are attached via a single PEG chain, for example, where two C105 muteins are attached to a polyethylene glycol (PEG) moiety, particularly PEG having a molecular weight of about 20,000.

Because it is possible that the inhibitory function of the preferred inhibitors is imparted by one or more discrete and separable portions, it is also envisioned that the therapeutic composition has as an active ingredient a portion or portions of the TNF inhibitor or IL-1 inhibitor that contol(s) interleukin-1 or TNF inhibition.

The therapeutic composition of the present invention can be administered parenterally by injection, although other effective administration forms, such as intraarticular injection, inhalant mists, orally active formulations, transdermal iontophoresis or suppositories, are also envisioned. One preferred carrier is physiological saline solution, but it is contemplated that other pharmaceutically acceptable carriers may also be used.

In one embodiment, it is envisioned that the carrier and the TNF inhibitor and the IL-1 inhibitor constitute a physiologically-compatible, slow-release formulation. The primary solvent in such a carrier can be either aqueous or non-aqueous in nature. In addition, the carrier can contain other pharmacologically-acceptable excipients for modifying or maintaining the pH, osmolarity, viscosity, clarity, color, sterility, stability, rate of dissolution, or odor of the formulation. Similarly, the carrier can contain still other pharmacologically-acceptable excipients for modifying or maintaining the stability, rate of dissolution, release, or absorption of the TNF inhibitor and/or IL-1 inhibitor. Such excipients are those substances usually and customarily employed to formulate dosages for parenteral administration in either unit dose or multi-dose form.

Once the therapeutic composition has been formulated, it can be stored in sterile vials as a solution, suspension, gel, emulsion, solid, or dehydrated or lyophilized powder. Such formulations may be stored either in a ready to use form or requiring reconstitution immediately prior to administration. The preferred storage of such formulations is at temperatures at least as low as 4°C and preferably at -70°C. It is also preferred that such formulations containing TNF inhibitor and IL-1 inhibitor are stored and administered at or near physiological pH. It is presently believed that administration in a formulation at a high pH (i.e. greater than 8) or at a low pH (i.e. less than 5) is undesirable.

Preferably, the manner of administering the formulations containing TNF inhibitor and IL-1 inhibitor for systemic delivery is via subcutaneous, intramuscular, intravenous, intranasal or vaginal or rectal suppository. Preferably the manner of administration of the formulations containing TNF inhibitor and IL-1 inhibitor for local delivery is via intraarticular, intratracheal, or instillation or inhalations to the respiratory tract. In addition it may be desirable to administer the TNF inhibitor and IL-1 inhibitor to specified portions of the alimentary canal either by oral administration of TNF inhibitor and IL-1 inhibitor in an appropriate formulation or device or by suppository or enema.

In an additional preferred mode for the treatment of TNF and IL-1 mediated diseases an initial intravenous bolus injection of TNF inhibitor and IL-1 inhibitor is administered followed by a continuous intravenous infusion of TNF inhibitor and IL-1 inhibitor.

The initiation of treatment for septic shock should be begun as soon as possible after septicemia or the chance of septicemia is diagnosed. For example, treatment may begun immediately following surgery or an accident or any other event that may carry the risk of initiating septic shock.

Preferred modes for the treatment of TNF or IL-1 mediated diseases and more particularly for the treatment of arthritis include: (1) a single intraarticular injection of TNF inhibitor and IL-1 inhibitor given periodically as needed to prevent or remedy flare up of arthritis; and (2) periodic subcutaneous injections of TNF inhibitor and IL-1 inhibitor.

Preferred modes for the treatment of TNF and IL-1 mediated diseases and more particularly for the treatment of adult respiratory distress syndrome include: 1) single or multiple intratracheal administrations of TNF inhibitor and IL-1 inhibitor, and 2) bolus or continuous intravenous infusion of TNF inhibitor and IL-1 inhibitor.

It is also contemplated that certain formulations containing TNF inhibitor and IL-1 inhibitor are to be administered orally. Preferably, TNF inhibitor and IL-1 inhibitor which is administered in this fashion is encapsulated. The encapsulated TNF inhibitor and IL-1 inhibitor may be formulated with or without those carriers customarily used in the compounding of solid dosage forms. Preferably, the capsule is designed so that the active portion of the formulation is released at that point in the gastro-intestinal tract when bioavailability is maximized and pre-systemic degradation is minimized. Additional excipients may be included to facilitate absorption of the TNF inhibitor and IL-1 inhibitor. Diluents, flavorings, low melting point waxes, vegetable oils, lubricants, suspending agents, tablet disintegrating agents, and binders may also be employed.

Regardless of the manner of administration, the specific dose is calculated according to the approximate body weight of the patient. In certain embodiments, the administration is designed to create a preselected concentration range of TNF inhibitor and IL-1 inhibitor in the patient's blood stream. It is believed that the maintenance of circulating concentrations of TNF inhibitor and IL-1 inhibitor of less than 0.01 ng per ml of plasma may not be an effective composition, while the prolonged maintenance of circulating levels in excess of 10 µg per ml may have undesirable side effects. Further refinement of the calculations necessary to determine the appropriate dosage for treatment involving each of the above mentioned formulations is routinely made by those of ordinary skill in the art and is within the gambit of tasks routinely performed by them without undue experimentation, especially in light of the dosage information and assays disclosed herein. These dosages may be ascertained through use of the established assays for determining dosages utilized in conjunction with appropriate dose-response data.

It should be noted that the TNF inhibitor and IL-1 inhibitor formulations described herein may be used for veterinary as well as human applications and that the term "patient should not be conned in a limiting manner. In the case of veterinary applications, the dosage ranges should be the same as specified above.

An interleukin-1 receptor antagonist (IL-1ra) and a tumor necrosis factor binding protein (TNFbp) have been developed for the treatment of inflammatory disease that are mediated by IL-1 and TNF. In two experimental systems, rheumatoid arthritis in rats and septic shock in baboons, blockade of the action of either IL-1 or TNF alone was sufficient to significantly inhibit the inflammatory response. In rodent arthritis, joint swelling was maximally inhibited by the administration alone of either IL-1ra or TNFbp in rats that were undergoing a reactivated arthritis induced by peptidoglycan-polysaccharide (PG/PS). In septic shock, baboons that were challenged with Escherichia coli were protected to a similar degree against lethality and hemodynamic alterations by the administration alone of either IL-1ra or TNFbp.

These results in experimental systems showing the beneficial effects of the administration of either inhibitor alone suggest that a synergistic interaction between IL-1 and TNF is desired to elicit a full pathologic response and that inhibition of the action of one of these key mediators is sufficient to maximally reduce the magnitude of the inflammatory process. It was believed that the single administration of either IL-1ra or TNFbp would be sufficient to maximally reduce inflammatory effects in other animal models of IL-1 and TNF-mediated diseases and that the combined administration of the inhibitors would afford no special advantage. Unexpectedly, however, treatment of rats undergoing an LPS-reactivated arthritis, an LPS-stimulated alveolitis, and an LPS-stimulated systemic inflammatory response with a combination of IL-1ra and TNFbp caused synergistic inhibitory effects on joint swelling, bronchoalveolar neutrophilic exudation and lethality, respectively. The examples below describe methods for treating IL-1- and TNF-mediated inflammatory diseases, such as rheumatoid arthritis, adult respiratory distress syndrome (ARDS) and sepsis, by combination therapy with IL-1ra and TNFbp.

The following examples are intended to illustrate, but not limit, the present invention.

### EXAMPLE I

This example demonstrates the synergistic effects of combination therapy with IL-1ra and TNFbp on LPS-induced reactivation of SCW-induced arthritis.

Current theories of the pathogenesis of rheumatoid arthritis and relaxed arthritides hypothesize two possible mechanisms of immunologically-mediated joint inflammation: (a) a microbial component that localizes to the joints, or (b) an articular autoantigen. Wilder, Experimental Animal Models of Chronic Arthritis. An animal model of rheumatoid arthritis induced by two microbial components (lipopolysaccharide (LPS) and peptidoglycan-polysaccharide (PG/PS) was used to investigate the use of combination therapy with the human recombinant 30kDa TNF inhibitor (hrTNF/inh30) and human recombinant IL-1 receptor antagonist (hrIL-1ra) for treatment of arthritis. According to R.L. Wilder in Immunopathogenetic Mechanisms of Arthritis, Chapter 9 entitled "Experimental Animal Models of Chronic Arthritis, "regarding streptococcal cell wall-induced arthritis, "the clinical, histological and radiological features of the experimental joint disease closely resemble those observed in adult and juvenile arthritis."

In the following experiments, the animal model described in Schwab, Experimental Medicine, 1688-1702, (1987), was used to induce arthritis in the tarsal joints of normal rats. Briefly, arthritis was induced by the sequential administration of two microbial components: (1) first streptococcal cell wall (SCW) products containing peptidoglycan-polysaccharide (PG/PS) were injected intraarticularly, and (2) twenty-one days later, lipopolysaccharide (LPS) from Salmonella typhimurium was injected intravenously.

### A. Experiment 1

Lewis rats, each weighing 140 to 160 grams, were injected intraarticularly (Charles River) into the ankle joint with SCW at a dose of 3 µg of rhamnose per joint Saline was injected into the contralateral joint to provide a control. The intraarticular injection of SCW caused an acute arthritis of relatively short duration with swelling of the joint peaking at one to two days post injection. After a period of twenty days, during which the acute inflammatory reaction resolved, lipopolysaccharide (LPS) was administered by intravenous injection at a dose of 45 µg per rat. Those dose of LPS was sufficient to reactive inflammation in the ankle joint previously injected with SCW and had little effect on the saline-injected ankle. To assess the extent of inflammation during the 72-hour period following the intravenous injection of LPS, the dimensions of the ankle joint were measured at 0, 24, 36, 48, and 72 hours after the reactivation of the arthritis.

The effects of IL-1ra and TNFbp when administered singly and in combination were tested on the development of joint swelling during the reactivation of the arthritis. The inhibitors and vehicle were administered subcutaneously at the nape of the neck at time 0, 2, 6, 12, 18, 24, 30, 36, and 42 hours relative to the intravenous injection of LPS. In the experiment, rats were treated as follows:
Group I - vehicle (sodium citrate, NaCl and EDTA)
Group II - IL-1ra (2 mg/kg)
Group III - TNFbp (1 mg/kg)
Group IV - IL-1ra (2 mg/kg) + TNFbp (1 mg/kg)

The maximum increases in joint diameter during the 72-hour period post reactivation are shown in Table 1. The results show that combination therapy with IL-1ra and TNFbp caused an additive inhibitory effect on LPS-reactivated arthritis. IL-1ra and TNFbp alone inhibited joint welling by 31% and 16% respectively, whereas combination therapy inhibited the swelling by 41%.

**TABLE 1**

| **Treatment Groups** | **Maximum Change in Joint Diameter**^{*****} **(mm)** | **Percentage Reduction** |
|---|---|---|
| Vehicle | 1.08 + 0.07 | |
| IL-1ra (2 mg/kg) | 0.74 + 0.08** | 16% |
| TNFbp (1 mg/kg) | 0.91 + 0.04 | 16% |
| IL-1ra (2 mg/kg) + TNFbp (1 mg/kg) | 0.59 + 0.11** | 41% |

| | | |
|---|---|---|
| * Values are means + standard error for 8 to 9 rats per group. The increases in joint diameter were calculated from the maximum swelling during the 72-hour period after the reactivation of the arthritis by the intravenous administration of LPS. | | |
| ** Significantly different than the vehicle group at p < 0.01 for the IL-1ra treated group and p < 0.005 for the IL-1ra + TNFbp treated group. | | |

### B. Experiment 2

In the second experiment, the inhibitors were administered again singly and in combination by subcutaneous injection at 0, 2, 6, 12, 18, 24, 30, 36, and 42 hours after LPS-induced reactivation. The dose of IL-1ra was reduced compared to that used in experiment 1, while the dose of TNFbp was unchanged. Rats were treated as follows:
Group I - vehicle
Group II - IL-1ra (0.1 mg/kg)
Group III - TNFbp (1 mg/kg)
Group IV - IL-1ra (0.1 mg/kg) + TNFbp (1 mg/kg)

Table 2 shows the time course of the changes in joint diameter of rats in groups I, II, III, and IV. Joint swelling of rats was reduced by combination therapy with IL-1ra and TNFbp as compared with the lack of effect on joint swelling of treatment with IL-1ra or TNFbp alone. The synergistic inhibitory effects of combination therapy are shown clearly in Figure 1 and Table 3. In Figure 1, the maximum increases in joint diameter during the 72-hour period after LPS-induced reactivation are shown. Combination therapy with IL-1ra and TNFbp caused a significant depression of joint swelling (35%) whereas treatment with either agent alone caused no significant reduction. In Table 3, the area under the curve {change diameter (mm) versus time (h)} for values listed in Table 2 are shown. During the 72 hour period of reactivation, combination therapy with IL-1ra and TNFbp caused a significant reduction in the overall joint swelling (53%) whereas treatment with either agent alone caused no significant reduction.

**TABLE 2**

| | **JOINT DIAMETER (mm) (mean ± S.E.)** | | | |
|---|---|---|---|---|
| **TIME PAST REACTIVATION (hours)** | **Vehicle** | **TNFbp 1 mg/kg** | **IL-1ra 0.1 mg/kg** | **TNFbp 1 mg/kg + IL-1ra 0.1 mg/kg** |
| 0 | 6.03 ± 0.03 | 6.39 ± 0.20 | 6.07 ± 0.03 | 6.20 ± 0.09 |
| 24 | 6.61 ± 0.06 | 6.70 ± 0.12 | 6.64 ± 0.08 | 6.49 ± 0.11 |
| 36 | 6.89 ± 0.10 | 7.03 ± 0.15 | 6.83 ± 0.10 | 6.62 ± 0.11 |
| 48 | 6.91 ± 0.09 | 6.91 ± 0.25 | 6.79 ± 0.03 | 6.76 ± 0.11 |
| 60 | 6.93 ± 0.10 | 7.14 ± 0.17 | 6.78 ± 0.05 | 6.67 ± 0.12 |
| 72 | 6.75 ± 0.13 | 6.88 ± 0.14 | 6.72 ± 0.08 | 6.65 ± 0.10 |
| n = 14 to 16 rats per group. | | | | |

**TABLE 3**

| | **AREA UNDER CURVE** | |
|---|---|---|
| | **Change in Joint Diameter vs. Time Past Reactivation mm vs. Time (Arbitrary Units)** | |
| I | Vehicle | 27.9 ± 3.0 |
| II | TNFbp (1 mg/kg) | 27.8 ± 2.6 |
| III | IL-1ra (0.1 mg/kg) | 28.4 ± 2.0 |
| IV | TNFbp (1 mg/kg) + IL-1ra (0.1 mg/kg) | 16.4 ± 2.2^{a}^{,}^{b}^{,}^{c} |

| | | |
|---|---|---|
| ^{a} IV vs I + = 3.05, p<0.005 | | |
| ^{b} IV vs II + = 3.34, p<0.005 | | |
| ^{c}IV vs III + = 3.98, p<0.001 | | |

The percentages reflecting the reduction of swelling with combination therapy were similar in experiments 1 and 2 (41% and 35 to 53% respectively). By taking advantage of the synergism in the inhibitory actions of IL-1ra and TNFbp on joint swelling, a similar level of therapeutic benefit in experiment 2 was achieved despite the ten-fold reduction in the dose of IL-1ra.

### EXAMPLE II

This example demonstrates the synergistic effects of combination therapy with IL-1ra and TNFbp on LPS-stimulated neutrophilic alveolitis as a method for treating Adult Respiratory Distress Syndrome (ARDS).

The experiment employed a septic stimulus, endotoxin, to induce acute, neutrophil-mediated pulmonary injury according to the model disclosed in Ulich, et al., American Journal of Pathology 138:1485-1496 (1991).

This model is briefly summarized as follows: endotoxin is injected intratracheally into the midcervical portion of the trachea of anesthetized rats. After a latent period of six hours, bronchoalveolar lavage (BAL) of the lungs is performed as a terminal procedure. Total and differential white blood cell counts are performed on the BAL fluid. Intratracheal injection of pyrogen-free saline yields BAL fluid with a predominance of alveolar macrophages (about 99%) in low numbers. Intratracheal injection of endotoxin causes a large increase in the number of BAL cells and a predominance of neutrophils. The acute neutrophilic influx into the alveolar space peaks at 6 to 12 hours and is accompanied by the accumulation of protein-containing edematous fluid into the alveolar spaces. IL-1 and TNF are present in the BAL fluid after stimulation with endotoxin. The alveolar macrophage is believed to be the source of cytokine synthesis. Moreover, intratracheal injection of exogenous IL-1 and TNF induces an acute intraalveolar neutrophilic exudate which is qualitatively similar to that induced by endotoxin.

Many animal models are used which approximate the altered pulmonary edema, sequestration of leukocytes, and hypoxemia during the acute phase of lung parenchymal injury during ARDS according to Murray et al., American Review of Respiratory Disease 138:720-723 (1991). The acute forms of ARDS occur in the presence of certain identifiable risk factors such as sepsis, aspiration, or multiple blood transfusions. Current investigations emphasize the central role of neutrophilic mediated injury in the pathophysiology of ARDS [Tate, Am. Rev. Resp. Dis. 128:552-559 (1983)]. Toxic products released by the activated neutrophil are believed to damage the alveolar capillary membrane. The permeability of damaged membrane is greatly increased stimulating the movement of plasma proteins and inflammatory cells into the alveolar spaces. In an experimental bovine model of ARDS of septic origin, neutrophil depletion protected against the development of pulmonary injury [Heflin, J. Clin. Invest. 68:1253-1260 (1981)].

A method for treating neutrophilic alveolitis in rats by the intratracheal administration of combination therapy with TNFbp (recombinant human 30kDa TNF inhibitor) and IL-1ra (recombinant human IL-1ra) is described. Lung injury was induced by the administration of endotoxin, lipopolysaccharide (LPS), from Salmonella typhimurium, at a dose of 5 µg per rat in a total volume of 0.5 ml of sterile PBS through a 27 gauge 1/2 inch needle inserted between tracheal rings in the surgically exposed midcervical region of the trachea. The inoculum was administered slowly into the trachea while monitoring the rate and depth of respiration of the rat. Six hours later, the rats were anesthetized with isoflurane so that a laparotomy could be performed in order to facilitate one lavage of the lungs. The caudal vena can was severed to decrease the blood content of the lungs. The diaphragm was opened to allow the lungs to expand during lavage. BAL was performed by injecting 40 ml of Hank's balanced salt solution into the bronchoalveolar spaces via an angiocath catheter, which was inserted and secured at the site of a midcervical tracheal incision. The inflammatory cellular influx was recovered from the pellet obtained by centrifuging the BAL fluid at 1500 rpm for 15 minutes. The total number of leukocytes was counted on a Coulter counter. The percentage of polymorphonuclear neutrophils was determined by performing a differential cell count manually on a slide of stained cells.

The effects of IL-1ra and TNFbp on the LPS-stimulated influx into the bronchoalveolar spaces were determined by administering the inhibitors intratracheally and simultaneously with the intratracheal instillation of LPS. TNF/inh was administered singly in doses ranging between 0.1 to 10 µg per rat. The results are summarized in Table 4. TNFbp at a dose of 0.1 µg per rat did not reduce the influx of neutrophils into the alveolar spaces. However, the intratracheal administration of TNF/inh at doses between 2.5 µg to 10 µg per rat caused a maximal reduction in the influx of neutrophils into the alveolar spaces in a range of 30 to 40%. IL-1ra was administered in doses ranging between 0.75 to 10 µg per rat (Table 5). IL-1ra at doses between 0.75 to 2.5 µg per rat did not reduce the influx of neutrophils into the alveolar spaces. However, the intratracheal administration of IL-1ra at doses of 5 and 10 µg per rat caused significant percentages of inhibition of 57% and 47%, respectively.

**TABLE 4**

| **Percentage Inhibition of Neutrophilic Influx into BAL Fluid** | |
|---|---|
| **Intracheal Dose of TNFbp (µg)** | **% Inhibition** |
| 0 | 0 |
| 0.1 | 10 ± 4 |
| 1.0 | 20 ± 7 |
| 2.5 | 35 ± 6 |
| 5 | 28 ± 8 |
| 7.5 | 33 ± 7 |
| 10 | 39 ± 7 |
| n = 4 to 8 per group. | |

**TABLE 5**

| **Percentage Inhibition of Neutrophilic Influx into BAL Fluid** | |
|---|---|
| **Intracheal Dose of IL-1ra (µg)** | **% Inhibition** |
| 0 | 0 |
| 0.75 | 0.1 ± 8 |
| 1 | 2 ± 9 |
| 2.5 | 1 ± 8 |
| 5 | 57 ± 16 |
| 10 | 40 ± 5 |
| n = 4 to 8 per group. | |

In a preliminary experiment, it was determined that combination therapy with maximally inhibitory doses of IL-1ra (100 µg/rat) and TNF/inh (10 µg per rat) caused an inhibitory effect on neutrophilic influx that was not significantly different than the effect of either agent alone.

In experiments 1 and 2, the effects of combination therapy was tested using submaximal or subthreshold doses of IL-1ra and TNF/inh on neutrophilic influx into the bronchoalveolar spaces. In experiment 1, IL-1ra and TNF/inh were given intratracheally simultaneously with LPS at the following doses:
Group I - Vehicle
Group II -TNF/inh (0.1 µg per rat)
Group III - IL-1ra (2.5 µg per rat)
Group IV-TNF/inh (0.1 µg per rat) + IL-1ra (2.5 µg per rat)

The number of neutrophils migrating into the bronchoalveolar spaces are shown in Table 6. The results demonstrated that combination therapy with IL-1ra and TNF/inh caused an additive inhibitory effect on LPS-stimulated neutrophilic alveolitis. TNF/inh and IL-1ra inhibited neutrophilic influx by 19 and 29%, respectively, whereas combination therapy inhibited the swelling by 47%.

**TABLE 6**

| **Treatment Groups** | **# Neutrophils in BAL fluid (X 10)** ***** | **Percentage Reduction** |
|---|---|---|
| Vehicle | 5.45 + 0.6 | |
| TNF/inh (0.1 µg/rat) | 4.43 + 0.9 | 19% |
| IL-1ra (2.5 µg/rat) | 3.89 + 0.3** | 29% |
| TNF/inh (0.1 µg/rat) + IL-1ra (2.5 µg/rat) | 2.88 + 0.5** | 47% |

| | | |
|---|---|---|
| * Values are means + standard error for 6 rats per group. | | |
| ** Significantly different than the vehicle group at p < 0.05 for the IL-1ra-treated group and p < 0.01 for the IL-1ra- and TNF/inh treated group. | | |

In experiment 2, the inhibitors (singly and in combination) were administered as in experiment 1 by an intratracheal route simultaneously with the LPS injury. Since IL-1ra in the previous experiment at 2.5 µg per rat caused a submaximal inhibitory effect on neutrophilic influx, the dose of IL-1ra was reduced to one that might elicit a subthreshold inhibitory effect. The objective was to manipulate the doses to a level at which the inhibitors singly would elicit no significant reduction, yet would synergize when administered in combination. The dose of TNF/inh was unchanged.
Group I - Vehicle
Group II - TNF/inh (0.1 µg per rat)
Group III - IL-1ra (0.75 µg per rat)
Group IV - TNF/inh (0.1 µg per rat)
   + IL-1ra (0.75 µg per rat)

Table 7 shows the numbers of neutrophils migrating into the bronchoalveolar spaces of rats in groups I, II, III, and IV. Neutrophilic influx was reduced by combination therapy with TNF/inh and IL-1ra as compared with the lack of effect on neutrophilic influx of treatments with TNF/inh and IL-1ra alone. The additive and synergistic inhibitory effects of combination therapy are clearly evident on acute lung injury. Combination therapy with IL-1ra and TNF/inh caused a significant reduction in neutrophilic influx of 40% whereas treatment with TNF/inh and IL-1ra alone caused insignificant reductions of neutrophilic influx of 12% and 7%, respectively.

**TABLE 7**

| | **Components** | **Neutrophils (10**^{**6**}**/lung)** | |
|---|---|---|---|
| I | LPS + vehicle | 5.10 ± 0.80 | (n = 8) |
| II | LPS + 0.1 µg TNFbp | 4.44 ± 0.40 | (n = 9) |
| III | LPS + 0.75 µg IL-1ra | 4.81 ± 0.70 | (n = 9) |
| IV | LPS + 0.1 µg TNFbp + 0.75 µg IL-1ra^{a}^{,}^{b}^{,}^{c} | 3.02 ± 0.30^{a}^{,b}^{,}^{c} | (n = 9) |

| | | | |
|---|---|---|---|
| ^{a} IV vs I + = 2.50, p<0.025 | | | |
| ^{b} IV vs II + = 2.82, p<0.025 | | | |
| ^{c} IV vs III + = 2.23, p<0.05 | | | |

### EXAMPLE III

IL-1ra and TNFbp, individually and in combination, were tested to determine their effects on enhancing survival time of animals challenged with a high dose of endotoxin to induce sepsis. In this set of experiments, rats (n=6 per group) were injected with 25 mg/kg of LPS according to the procedure of Example II. Pegylated TNFbp (the C105 mutein of the 30kDa TNF inhibitor pegylated with polyethylene-20K) or vehicle alone was injected intravenously and simultaneously with LPS, while IL-1ra was injected subcutaneously at 0, 4, 8, 12 and 18 hours at the doses identified in Table 8. Final survival assessments were made at 96 hours after the administration of endotoxin.

The survival percentages for each test group are also provided in Table 8. The results demonstrate that the combination of IL-1ra and TNFbp produced a synergistic enhancement of survival, whereas IL-1ra or TNFbp alone was not fully protective.

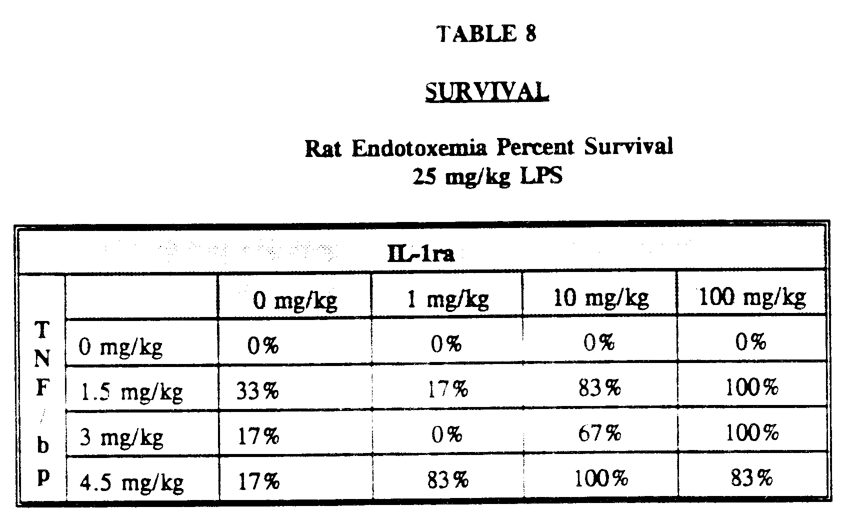

### EXAMPLE IV

It is believed that elevated corticosterone levels associated with certain conditions, such as sepsis and burn patients for example, contribute significantly to the immunosuppression and cachectic effect observed in these conditions. The effects of IL-1ra and TNFbp, individually and in combination, on serum corticosterone levels were determined on endotoxin-induced sepsis in rats.

Lewis rats were challenged with 10 mg/kg LPS according to the procedure of Example II. The pegylated TNFbp of Example III (1.5 mg/kg) was injected intravenously and simultaneously with LPS, while hrIL-1ra (100 mg/kg) was injected subcutaneously at 0, 4, 8, 12 and 18 hours after the administration of endotoxin.

Serum corticosterone levels were tested at 24 hours by radioimmunoassay using the corticosterone-³H kit (ICN Biomedicals. Inc., Costa Mesa, California) according to the manufacturer's instructions. Table 9 summarizes the results of the assays.

**TABLE 9**

| **Number of Animals** | **Components** | **Serum Corticosterone Levels (ng/ml) + S.E.** |
|---|---|---|
| 4 | Vehicle (no LPS) | 164 ± 62 |
| 4 | Vehicle + LPS | 750 ± 49 |
| 8 | IL-1ra + LPS | 279 ± 54 |
| 8 | TNFbp + LPS | 489 ± 43 |
| 8 | IL-1ra + TNFbp + LPS | 103 ± 25^{a}^{,}^{b}^{,}^{c} |

| | | |
|---|---|---|
| ^{a}t = 2.95, p<.025 (combination vs. IL-1ra + LPS) | | |
| ^{b}t = 7.85, p<.001 (combination vs. TNFbp + LPS) | | |
| ^{c}t = 13.30, p<0.001 (combination vs. vehicle + LPS) | | |

As shown in Table 9, the corticosterone level was significantly reduced in animals receiving the combination of IL-1ra and TNFbp compared to those receiving IL-1ra or TNFbp alone. The combination therapy demonstrated that together IL-1ra and TNFbp caused an additive effect on the reduction of serum corticosterone level. These results suggest that the combination therapy may have anti-immunosuppressive and anti-cachectic effects resulting in a therapeutically beneficial method for the treatment of sepsis and burn patients.

### EXAMPLE V

Platelet counts were also tested on rats injected with endotoxin to induce endotoxemia. It is known that platelets are consumed during endotoxemia resulting in a significant decrease in platelets.

Lewis rats were challenged with 10 mg/kg LPS according to the procedure of Example II. The pegylated TNFbp of Example III (1.5 mg/kg) was injected intravenously into each rat simultaneously with LPS, while hrIL-1ra (100 mg/kg) was injected subcutaneously at 0, 4, 8, 12 and 18 hours after the administration of endotoxin.

Platelet counts were measured at 24 hours according to standard procedures known in the art (automated counter based on particle size). Table 10 summarizes the results of the assays.

**TABLE 10**

| | **EXPERIMENT NO.1** | | **EXPERIMENT NO.2** | |
|---|---|---|---|---|
| **Component(s)** | **Platelet Levels**^{**a**} | **Number of Animals** | **Platelet Levels**^{**b**} | **Number of Animals** |
| Vehicle (no LPS) | 825 ± 23 | 5 | 762 ± 14 | 4 |
| Vehicle + LPS | 31 ± 5 | 13 | 19 ± 4 | 4 |
| IL-1ra + LPS | 20 ± 1 | 10 | 29 ± 3 | 8 |
| TNFbp + LPS | 28 ± 6 | 12 | 23 ± 3 | 8 |
| IL-1ra + TNFbp + LPS | 64 ± 8^{a}^{,}^{b}^{,}^{c} | 11 | 44 ± 4^{d}^{,}^{e}^{,}^{f} | 8 |
| Platelet levels = 10³/µl ± S.E. | | | | |

| | | | | |
|---|---|---|---|---|
| ^{a}t = 5.10, p<0.001 (combination vs. IL-1ra + LPS) | | | | |
| ^{b}t = 2.67, p<0.005 (combination vs. TNFbp + LPS) | | | | |
| ^{c}t = 3.67, p<0.005 (combination vs. vehicle + LPS) | | | | |
| ^{d}t = 2.94, p<0.025 (combination vs. IL-1ra + LPS) | | | | |
| ^{e}t = 4.25, p<0.001 (combination vs. TNFbp + LPS) | | | | |
| ^{f}t = 3.87, p<0.005 (combination vs. vehicle + LPS) | | | | |

In both experiments, the combination treatment value is significantly different than values for IL-1ra or TNFbp treatment alone. These results suggest that the combination therapy reduces the consumption of platelets during endotoxemia.

## Claims

1. A pharmaceutical composition comprising IL-1 inhibitor IL-1ra or an IL-1 inhibitory portion or mutein thereof and a TNF inhibitor in a pharmaceutically acceptable carrier.

2. The pharmaceutical composition of claim 1, wherein said IL-1ra comprises at least one compound from the group consisting of: IL-1raα, IL-1raβ and IL-1rax or an IL-1 inhibitory portion of mutein thereof.

3. The pharmaceutical composition of claim 1, wherein said TNF-inhibitor comprises at least one compound selected from the group consisting of: 30kDa TNF inhibitor or a TNF inhibitory fragment or mutein thereof; 40kDa TNF inhibitor or a TNF inhibitory fragment or mutein thereof; 40kDa TNF inhibitor Δ51; and 40kDa TNF inhibitor Δ53.

4. The pharmaceutical composition of claim 1, wherein said IL-1 inhibitor is human recombinant IL-1ra and said TNF inhibitor is human recombinant 30kDa TNF inhibitor.

5. The pharmaceutical composition of any one of claims 1-4, wherein said pharmaceutically acceptable carrier comprises a physiologically-compatible, slow-release formulation.

6. The pharmaceutical composition of any one of claims 1-5, wherein said IL-1ra is attached to one or more repeating polymeric moieties, including polyethylene glycol (PEG).

7. The pharmaceutical composition of any one of claims 1-6, wherein said TNF inhibitor is the 30kDa TNF inhibitor and has a cysteine at position 105.

8. The pharmaceutical composition of any one of claims 1 to 7, wherein said TNF inhibitor is attached to one or more repeating polymeric moieties, including polyethylene glycol (PEG).

9. A pharmaceutical composition comprising IL-1 inhibitor IL-1ra or an IL-1 inhibitory portion or mutein thereof and a TNF inhibitor as a combined preparation for simultaneous, separate or sequential use in treatment or prevention of TNF-mediated diseases or IL-1 mediated diseases.

10. Use of IL-1 inhibitor IL-1ra or an IL-1 inhibitory portion or mutein thereof and a TNF-inhibitor for the preparation of a medicament for the treatment or prevention of TNF-mediated diseases or IL-1 mediated diseases, wherein the IL-1 inhibitor IL-1ra or an IL-1 inhibitory portion or mutein thereof and the TNF inhibitor can be administered simultaneously, separately or sequentially.

11. Use according to claim 10, wherein said IL-1ra comprises at least one compound from the group consisting of: IL-1raα, IL-1raβ and IL-1rax or an IL-1 inhibitory portion or mutein thereof.

12. Use according to claim 10, wherein said TNF-inhibitor comprises at least one compound selected from the group consisting of: 30kDa TNF inhibitor or a TNF inhibitory fragment or mutein thereof; 40kDa TNF inhibitor or a TNF inhibitory fragment or mutein thereof 40kDa TNF inhibitor Δ51 and 40kDa TNF inhibitor Δ53.

13. Use according to claim 10, wherein said TNF inhibitor is human recombinant 30kDa TNF inhibitor.

14. Use according to claim 10, wherein said IL-1 inhibitor is human recombinant IL-1ra.

15. Use according to any one of claims 10 to 14, wherein said disease is selected from the group comprising of arthritis, inflammatory bowel disease, sepsis, septic shock, ischemia injury, reperfusion injury, osteoporosis, asthma, insulin diabetes, myelogenous and other leukemias, psoriasis, adult respiratory distress syndrome, cachexia/anorexia, and pulmonary fibrosis.

16. Use of any one of claims 10-15, wherein said IL-1ra and TNF inhibitor are in a pharmaceutically acceptable carrier for administration.

17. Use of claim 16, wherein said pharmaceutically acceptable carrier comprises a physiologically-compatible, slow-release formulation.

18. Use of any one of claims 10-17, wherein said IL-1ra and TNF inhibitor are for administration by continuous intravenous infusion.

19. Use according to any one of claims 10-18, wherein said IL-1ra is substantially pure.

20. Use according to any one of claims 10-19, wherein said IL-1ra is glycosylated or wherein said IL-1ra is not glycosylated.

21. Use according to any one of claims 10-20, wherein said IL-1ra has a methionine residue at the N-terminus.

22. Use of any one of claims 10-21, wherein said IL-1ra is attached to one or more repeating polymeric moieties, including polyethylene glycol.

23. Use of claims 22, wherein the repeating polymeric moiety is polyethylene glycol.

24. Use according to any one of claims 10-23, wherein the IL-1ra is for intra-articular, subcutaneous, intramuscular, intravenous, intranasal, or oral administration.

25. Use according to any one of claims 10-24, wherein said TNF inhibitor is the 30kDa TNF inhibitor has a cysteine at position 105.

26. Use according to any one of claims 10-25, wherein said TNF inhibitor is substantially pure.

27. Use according to any one of claims 10-26, wherein the TNF inhibitor is glycosylated or wherein said TNF inhibitor is not glycosylated.

28. Use according to any one of claims 10-27, wherein the TNF inhibitor has a methionine residue at the N-terminus.

29. Use according to any one of claims 10-28, wherein said TNF inhibitor and IL-1 inhibitor are attached to one or more repeating polymeric moieties, including polyethylene glycol.

30. Use of claim 29, wherein the repeating polymeric moiety is polyethylene glycol.

31. Use according to any one of claims 10-30, wherein the TNF inhibitor is for intra-articular, subcutaneous, intramuscular, intravenous, intranasal, or oral administration.

## Patentansprüche

1. Eine pharmazeutische Zusammensetzung, die den IL-1-Inhibitor IL-1ra oder einen IL-1-inhibitorischen Teil oder ein Mutein davon und einen TNF-Inhibitor in einem pharmazeutisch verträglichen Träger umfaßt.

2. Pharmazeutische Zusammensetzung nach Anspruch 1, bei der IL-1ra mindestens eine Verbindung aus der folgenden Gruppe umfaßt: IL-1raα, IL-1raβ und IL-1rax, oder ein IL-1-inhibitorischer Teil oder ein Mutein davon.

3. Pharmazeutische Zusammensetzung nach Anspruch 1, bei der der TNF-Inhibitor mindestens eine Verbindung umfaßt, die aus der folgenden Gruppe ausgewählt wird: 30 kDa TNF-Inhibitor oder ein TNF-inhibitorisches Fragment oder Mutein davon, 40 kDa TNF-Inhibitor oder ein TNF-inhibitorisches Fragment oder Mutein davon, 40 kDa TNF-Inhibitor Δ51 und 40 kDa TNF-Inhibitor Δ53.

4. Pharmazeutische Zusammensetzung nach Anspruch 1, bei der der IL-1-Inhibitor rekombinanter menschlicher IL-1ra ist und der TNF-Inhibitor rekombinanter menschlicher 30 kDa TNF-Inhibitor ist.

5. Pharmazeutische Zusammensetzung nach einem der Ansprüche 1 - 4, bei der der pharmazeutisch verträgliche Träger eine physiologisch kompatible Formulierung zur verzögerten Freigabe umfaßt.

6. Pharmazeutische Zusammensetzung nach einem der Ansprüche 1 - 5, bei der IL-1ra an eine oder mehrere repetitive Polymereinheiten, einschließlich Polyethylenglykol (PEG), gekoppelt ist.

7. Pharmazeutische Zusammensetzung nach einem der Ansprüche 1 - 6, bei der der TNF-Inhibitor der 30 kDa TNF-Inhibitor ist und an der Position 105 ein Cystein aufweist.

8. Pharmazeutische Zusammensetzung nach einem der Ansprüche 1 - 7, bei der der TNF-Inhibitor an eine oder mehrere repetitive Polymereinheiten, einschließlich Polyethylenglykol (PEG), gekoppelt ist.

9. Pharmazeutische Zusammensetzung, die den IL-1-Inhibitor IL-1ra oder einen IL-1-inhibitorischen Teil oder ein Mutein davon und einen TNF-Inhibitor umfaßt, als kombiniertes Präparat zur simultanen, separaten oder sequentiellen Anwendung bei der Behandlung oder Vorbeugung von TNF-vermittelten Krankheiten oder IL-1-vermittelten Krankheiten.

10. Verwendung des IL-1-Inhibitors IL-1ra oder eines IL-1-inhibitorischen Teils oder eines Muteins davon und eines TNF-Inhibitors zur Herstellung eines Arzneimittels zur Behandlung oder Vorbeugung von TNF-vermittelten Krankheiten oder IL-1-vermittelten Krankheiten, wobei der IL-1-Inhibitor IL-1ra oder ein IL-1-inhibitorischer Teil oder ein Mutein davon und der TNF-Inhibitor simultan, separat oder sequentiell verabreicht werden können.

11. Verwendung nach Anspruch 10, wobei IL-1ra mindestens eine Verbindung der folgenden Gruppe umfaßt: IL-1raα, IL-1raβ und IL-1rax, oder ein IL-1-inhibitorischer Teil oder ein Mutein davon.

12. Verwendung nach Anspruch 10, wobei der TNF-Inhibitor mindestens eine Verbindung umfaßt, die aus der folgenden Gruppe ausgewählt wird: 30 kDa TNF-Inhibitor oder ein TNF-inhibitorisches Fragment oder Mutein davon, 40 kDa TNF-Inhibitor oder ein TNF-inhibitorisches Fragment oder Mutein davon, 40 kDa TNF-Inhibitor Δ51 und 40 kDa TNF-Inhibitor Δ53.

13. Verwendung nach Anspruch 10, wobei der TNF-Inhibitor rekombinanter menschlicher 30 kDa TNF-Inhibitor ist.

14. Verwendung nach Anspruch 10, wobei der IL-1-Inhibitor rekombinanter menschlicher IL-1ra ist.

15. Verwendung nach einem der Ansprüche 10 - 14, wobei die Krankheit aus folgender Gruppe ausgewählt wird: Arthritis, entzündliche Darmkrankheit, Sepsis, septischer Schock, ischämische Schädigung, Reperfusionsschädigung, Osteoporose, Asthma, Insulinmangeldiabetes, myeloische Leukämie und andere Leukämien, Psoriasis, Atemnotsyndrom bei Erwachsenen, Kachexie/Anorexie und Lungenfibrose.

16. Verwendung nach einem der Ansprüche 10 - 15, wobei IL-1ra und der TNF-Inhibitor zur Verabreichung in einem pharmazeutisch verträglichen Träger vorliegen.

17. Verwendung nach Anspruch 16, wobei der pharmazeutisch verträgliche Träger eine physiologisch kompatible Formulierung zur verzögerten Freigabe umfaßt.

18. Verwendung nach einem der Ansprüche 10 - 17, wobei IL-1ra und der TNF-Inhibitor zur Verabreichung durch kontinuierliche intravenöse Infusion sind.

19. Verwendung nach einem der Ansprüche 10 - 18, wobei IL-1ra im wesentlichen rein ist.

20. Verwendung nach einem der Ansprüche 10 - 19, wobei IL-1ra glykosyliert oder IL-1ra nicht glykosyliert ist.

21. Verwendung nach einem der Ansprüche 10 - 20, wobei IL-1ra am N-Terminus einen Methionin-Rest aufweist.

22. Verwendung nach einem der Ansprüche 10 - 21, wobei IL-1ra an eine oder mehrere repetitive Polymereinheiten, einschließlich Polyethylenglykol, gekoppelt ist.

23. Verwendung nach Anspruch 22, wobei die repetitive Polymereinheit Polyethylenglykol ist.

24. Verwendung nach einem der Ansprüche 10 - 23, wobei IL-1ra zur intraartikulären, subkutanen, intramuskulären, intravenösen, intranasalen oder oralen Verabreichung ist.

25. Verwendung nach einem der Ansprüche 10 - 24, wobei der TNF-Inhibitor der 30 kDa TNF-Inhibitor ist und an der Position 105 ein Cystein aufweist.

26. Verwendung nach einem der Ansprüche 10 - 25, wobei der TNF-Inhibitor im wesentlichen rein ist.

27. Verwendung nach einem der Ansprüche 10 - 26, wobei der TNF-Inhibitor glykosyliert oder der TNF-Inhibitor nicht glykosyliert ist.

28. Verwendung nach einem der Ansprüche 10 - 27, wobei der TNF-Inhibitor am N-Terminus einen Methionin-Rest aufweist.

29. Verwendung nach einem der Ansprüche 10 - 28, wobei der TNF-Inhibitor und der IL-1-Inhibitor an eine oder mehrere repetitive Polymereinheiten, einschließlich Polyethylenglykol, gekoppelt sind.

30. Verwendung nach Anspruch 29, wobei die repetitive Polymereinheit Polyethylenglykol ist.

31. Verwendung nach einem der Ansprüche 10 - 30, wobei der TNF-Inhibitor zur intraartikulären, subkutanen, intramuskulären, intravenösen, intranasalen oder oralen Verabreichung ist.

## Revendications

1. Composition pharmaceutique comprenant l'inhibiteur d'IL-1 IL-1ra ou l'une de ses parties inhibitrices d'IL-1 ou l'une de leurs mutéines et un inhibiteur de TNF, dans un véhicule pharmaceutiquement acceptable.

2. Composition pharmaceutique de la revendication 1, dans laquelle ledit IL-1ra comprend au moins un composé compris dans l'ensemble consistant en : IL-1raα, IL-1raβ et IL-1rax ou une de leurs parties inhibitrices d'IL-1 ou de leurs mutéines.

3. Composition pharmaceutique de la revendication 1, dans lequel ledit inhibiteur de TNF comprend au moins composé choisi dans l'ensemble consistant en : inhibiteur de TNF de 30 kDa ou l'un de ses fragments inhibiteurs de TNF ou de ses mutéines ; inhibiteur de TNF de 40 kDa ou l'un de ses fragments inhibiteurs de TNF ou de ses mutéines ; inhibiteur de TNF de 40 kDa Δ51 ; et inhibiteur de TNF de 40 kDa Δ53.

4. Composition pharmaceutique de la revendication 1, dans laquelle ledit inhibiteur d'IL-1 est IL-1ra recombinant humain et ledit inhibiteur de TNF est un inhibiteur de TNF recombinant humain de 30 kDa.

5. Composition pharmaceutique de l'une quelconque des revendications 1-4, dans laquelle ledit véhicule pharmaceutiquement acceptable comprend une formulation physiologiquement compatible à libération lente.

6. Composition pharmaceutique de l'une quelconque des revendications 1-5, dans laquelle ledit IL-1ra est attaché à un ou plusieurs parties polymères répétés, incluant polyéthylèneglycol (PEG).

7. Composition pharmaceutique de l'une quelconque des revendications 1-6, dans laquelle ledit inhibiteur de TNF est un inhibiteur de TNF de 30 kDa et comprend un résidu cystéine en position 105.

8. Composition pharmaceutique de l'une quelconque des revendications 1 à 7, dans laquelle ledit inhibiteur de TNF est attaché à une ou plusieurs parties polymères répétées, incluant le polyéthylèneglycol (PEG).

9. Composition pharmaceutique comprenant l'inhibiteur d'IL-1 IL-1ra ou l'une de ses parties inhibitrices d'IL-1 ou l'une de leurs mutéines et un inhibiteur de TNF sous forme de préparation combinée pour une utilisation simultanée, séparée ou séquentielle dans le traitement ou la prévention de maladies médiées par TNF ou de maladies médiées par IL-1.

10. Utilisation d'un inhibiteur d'IL-1 IL-1ra ou l'une de ses parties inhibitrices d'IL-1 ou l'une de leurs mutéines et un inhibiteur de TNF pour la préparation d'un médicament destiné au traitement ou à la prévention de maladies médiées par TNF ou de maladies médiées par IL-1, où l'inhibiteur d'IL-1 IL-1ra ou l'une de ses parties inhibitrices d'IL-1 ou l'une de leurs mutéines et l'inhibiteur de TNF peuvent être administrés simultanément, séparément ou séquentiellement.

11. Utilisation selon la revendication 10, dans laquelle ledit IL-1ra comprend au moins un composé compris dans l'ensemble consistant en : IL-1raα, IL-1raβ et IL-1rax ou une de ses parties inhibitrices d'IL-1 ou de leurs mutéines.

12. Utilisation selon la revendication 10, dans lequel ledit inhibiteur de TNF comprend au moins un composé choisi dans l'ensemble consistant en : inhibiteur de TNF de 30 kDa ou l'un de ses fragments inhibiteurs de TNF ou l'une de leurs mutéines ; inhibiteur de TNF de 40 kDa ou l'un de ses fragments inhibiteurs de TNF ou l'une de leurs mutéines ; inhibiteur de TNF de 40 kDa Δ51 ; et inhibiteur de TNF de 40 kDa Δ53.

13. Utilisation selon la revendication 10, dans laquelle ledit inhibiteur de TNF est un inhibiteur de TNF humain recombinant de 30 kDa.

14. Utilisation selon la revendication 10, dans laquelle ledit inhibiteur d'IL-1 est l'IL-1ra recombinant humain.

15. Utilisation selon l'une quelconque des revendications 10 à 14, dans laquelle ladite maladie est choisie dans l'ensemble consistant en : arthrite, maladie inflammatoire du tube digestif, septicémie, choc septique, lésion ischémique, lésion de reperfusion, ostéoporose, asthme, diabète insulinique, leucémies myélogènes et autres, psoriasis, syndrome de détresse respiratoire de l'adulte, cachexie/anorexie, et fibrose pulmonaire.

16. Utilisation de l'une quelconque des revendications 10-15, dans laquelle IL-1ra et l'inhibiteur de TNF sont dans un véhicule pharmaceutiquement acceptable pour administration.

17. Utilisation de la revendication 16, dans laquelle ledit véhicule pharmaceutiquement acceptable comprend une formulation physiologiquement compatible à libération lente.

18. Utilisation selon l'une quelconque des revendications 10-17, dans laquelle lesdits IL-1ra et inhibiteur de TNF sont destinés à une administration par perfusion intraveineuse continue.

19. Utilisation selon l'une quelconque des revendications 10-18, dans laquelle ledit IL-1ra est essentiellement pur.

20. Utilisation selon l'une quelconque des revendications 10-19, dans laquelle ledit IL-1ra est glycosylé ou dans laquelle ledit IL-1ra n'est pas glycosylé.

21. Utilisation selon l'une quelconque des revendications 10-20, dans laquelle ledit IL-1ra comprend un résidu méthionine à l'extrémité N-terminale.

22. Utilisation selon l'une quelconque des revendications 10-21, dans laquelle ledit IL-1ra est attaché à une ou plusieurs parties polymères répétées, incluant le polyéthylèneglycol.

23. Utilisation de la revendication 22, dans laquelle ladite partie polymère répétée est du polyéthylèneglycol.

24. Utilisation selon l'une quelconque des revendications 10-23, dans laquelle ledit IL-1ra est destiné à une administration intra-articulaire, sous-cutanée, intramusculaire, intraveineuse, intranasale, ou orale.

25. Utilisation selon l'une quelconque des revendications 10-24, dans laquelle ledit inhibiteur de TNF est un inhibiteur de TNF de 30 kDa et comprend un résidu cystéine en position 105.

26. Utilisation selon l'une quelconque des revendications 10-25, dans laquelle ledit inhibiteur de TNF est essentiellement pur.

27. Utilisation selon l'une quelconque des revendications 10-26, dans laquelle l'inhibiteur de TNF est glycosylé ou dans laquelle l'inhibiteur de TNF n'est pas glycosylé.

28. Utilisation selon l'une quelconque des revendications 10-27, dans laquelle ledit inhibiteur de TNF comprend un résidu méthionine à l'extrémité N-terminale.

29. Utilisation selon l'une quelconque des revendications 10-28, dans laquelle ledit inhibiteur de TNF et ledit inhibiteur d'IL-1 est attaché à une ou plusieurs parties polymères répétées, incluant le polyéthylèneglycol.

30. Utilisation de la revendication 29, dans laquelle ladite partie polymère répétée est du polyéthylèneglycol.

31. Utilisation selon l'une quelconque des revendications 10-30, dans laquelle ledit inhibiteur de TNF est destiné à une administration intra-articulaire, sous-cutanée, intramusculaire, intraveineuse, intranasale, ou orale.
